# EUROPEAN PATENT SPECIFICATION

(11) **EP 1 458 666 B1**
(45) Date of publication and mention of the grant of the patent: **30.08.2006**
(21) Application number: 02752283.8
(22) Date of filing: 12.07.2002
(51) Int. Cl.: C07C 47/225, A61Q 13/00, A61Q 15/00, C11D 3/50, A23L 1/226, C07C 47/293, C07C 47/395, C07D 333/22

(54) **IMPROVED AROMACHEMICALS**
VERBESSERTE GERUCHSSTOFFE
GENERATEURS D'AROMES AMELIORES

(30) Priority: 19.12.2001 US 342150 P; 15.01.2002 US 348580 P; 03.05.2002 US 377914 P; 17.06.2002 US 389298 P; 02.07.2002 US 355052 P
(43) Date of publication of application: 22.09.2004
(60) Divisional application: 06076289.5
(73) Proprietor: Flexitral, Inc., Chantilly, VA 20151 (US)
(72) Inventor: TURIN, Luca, London NW1 7NB (GB)
(74) Representative: Crowhurst, Charlotte Waveney
(86) International application number: PCT/US2002/022120
(87) International publication number: WO 2003/053902

(56) References cited:
- EP-A- 0 219 146
- EP-A- 0 418 680
- EP-A- 0 801 049
- WO-A-01/06853
- WO-A1-03/053901
- DE-A- 2 617 816
- FR-A- 1 393 451
- FR-A- 2 543 134
- GB-A- 1 082 364
- US-A- 3 770 836
- US-A- 3 950 429
- US-A- 4 097 531
- US-A- 4 151 103
- US-A- 4 435 428
- US-A- 4 521 331
- US-A- 4 536 583
- US-A- 4 658 067
- US-A- 6 051 548
- MASAKI Y ET AL: "Substrate-specific rearrangement and acetonidation of epoxy-ethers catalyzed by tetracyanoethylene" CHEMISTRY LETTERS, no. 1, January 1993 (1993-01), pages 17-20, XP002224829
- ARMSTRONG A ET AL: "Intramolecular epoxidation in unsaturated ketones and oxaziridines" JOURNAL OF THE CHEMICAL SOCIETY, PERKIN TRANSACTIONS 1, no. 21, 1 November 2001 (2001-11-01), pages 2861-2873, XP002224830
- SCHULTE-ELTE K H ET AL: "Photooxygenation of 3,3-dialkylsubstituted allyl alcohols. Occurence of syn preference in the ene addition of (1)O2 at E/Z-isomeric allyl alcohols" HELVETICA CHIMICA ACTA, vol. 62, no. 3, 20 April 1979 (1979-04-20), pages 816-829, XP002224831
- SCHELLER M E ET AL: "Syntheses of cyclopropyl silyl ketones" HELVETICA CHIMICA ACTA, vol. 68, no. 1, 5 February 1986 (1986-02-05), pages 44-52, XP002224832
- RICKARDS R W ET AL: "Synthesis of four stereoisomers of the higher dipteran juvenile hormone III bisepoxide" TETRAHEDRON LETTERS, vol. 33, no. 52, 22 December 1992 (1992-12-22), pages 8137-8140, XP002224833
- CALµ V ET AL: "Enantiomeric selection via 1,3-elimination. A simultaneous kinetic resolution of halohydrins and epoxides" TETRAHEDRON LETTERS, no. 49, 1978, pages 4963-4966, XP002224834
- ZIEGLER F E ET AL: "Carbon-Carbon Bond Forming Reactions with Oxiranyl Radicals" TETRAHEDRON LETTERS, vol. 37, no. 35, 26 August 1996 (1996-08-26), pages 6299-6302, XP004030674
- MORI N ET AL: "Synthesis of (2R,3R)-Epoxyneral, a sex pheromone of the acarid mite, Caloglyphus sp. (Astigmata: Acaridae)" TETRAHEDRON LETTERS, vol. 36, no. 9, 27 February 1995 (1995-02-27), pages 1477-1478, XP004028605
- SAKAGUCHI S ET AL: "Selective oxidation of monoterpenes with hydrogen peroxide catalyzed by peroxotungstophosphate (PCWP)" JOURNAL OF ORGANIC CHEMISTRY, vol. 61, no. 16, 9 August 1996 (1996-08-09), pages 5307-5311, XP002224835
- FILLIATRE C ET AL: "Synthèse de dérivés cyclopropaniques en série p-menthénique" COMPTES RENDUS HEBDOMADAIRES DES SEANCES DE L'ACADEMIE DES SCIENCES, SERIE C, vol. 273, 18 October 1971 (1971-10-18), pages 1001-1004, XP002225027
- JULIA M ET AL: "Préparation de composés terpéniques et apparentés, à partir de méthyl cyclopropyl cétone" BULLETIN DE LA SOCIETE CHIMIQUE DE FRANCE, 1960, pages 1072-1079, XP002225028
- TABER D.F. ET AL: 'Synthesis of (-)-delobanone' JOURNAL OF ORGANIC CHEMISTRY vol. 66, no. 10, 18 May 2001, pages 3423 - 3426
- UENISHI J. ET AL: 'An extremely mild desulfurization of thiiranes; an efficient transformation from geraniol to (+)- and (-)-linalool' TETRAHEDRON LETTERS vol. 35, no. 36, 05 September 1994, pages 6697 - 6700
- MATURA M. ET AL: 'Selected oxidized fragrance terpenes are common allergens' CONTACT DERMTITIS vol. 52, 2005, pages 320 - 328
- LARSEN W. ET AL: 'Fragrance contact dermatitis: a worldwide multicenter investigation (Part II)' CONTACT DERMATITIS vol. 44, 2001, pages 344 - 346
- "Opinion of the Scientific Committee on Cosmetic Products and Non-food Products intended for Consumers concerning Linalool, SCCNFP/0760/03" 9 December 2003 (2003-12-09),
- SKÖLD M. ET AL: 'Contact allergens formed on air exposure of linalool. Identification and quantification of primary and secondary oxidation products and the effect on skin sensitization' CHEMICAL RESEARCH IN TOXICOLOGY vol. 17, 2004, pages 1697 - 1705
- SKÖLD M. ET AL: 'Studies on the autoxidation and sensitizing capacity of fragrance chemical linalool, identifying a linalool hydroperoxide' CONTACT DERMATITIS vol. 46, 2002, pages 267 - 272
- BAUER K. ET AL: 'Common Fragrance and Flavor Materials', 1985, VCH, WEINHEIM pages 18-20 - 22-23

## Description

### FIELD OF THE INVENTION

The present invention relates generally to the field of flavorings and fragrances. More particularly, the present invention relates to perfumes and other fragrant articles based on aromachemicals which overcome the stability limitations and/or allergic nature of the native compounds.

### BACKGROUND OF THE INVENTION

Many aromachemicals are used in the flavoring and fragrance industries. For example, citral has a lemon scent and as such is used as a flavor and/or fragrance in many articles of manufacture. However, many aromachemicals include double bonds, aldehyde groups and other reactive groups which are potentially susceptible to reaction and may result in a limited useful lifetime. Further, many essential oil fragrances have recently been determined to cause allergic reactions, and it is becoming increasingly difficult to bring such compounds to market.

Many aromachemicals, which are fundamental to the formation of various fragrances have been placed on the allergens list and are being banned or restricted in many commercial regions. The bans or restrictions will undoubtedly have a considerable effect on the quality of various fragrances, largely because the reduction in the perfumers palette makes the creation of certain notes virtually impossible. Examples of aromachemicals used to form muguet accords include hydroxycitronellal, lyral (IFF) lilial, and bourgeonal. The only lily-of-the-valley materials left to the perfumer are the cruder odorants like cyclamen aldehyde, majantol (Haannan &Reimer), mayol (Firmenich), and the newer dupical (Quest), elintael (Quest), florahydral (Givaudan-Roure). Even rose notes will become difficult to create with both geraniol and citronellol on the list, and many other classical and fantasy odour notes and themes will become difficult to achieve, with the perfumer being effectively hand-cuffed.

Linalool and citronellol are two compounds frequently used in perfumery, see K. Bauer and D. Garbe, "Common Fragrance and Flavor Materials", VCH, Weinheim, 1985, pages 18-20, 22 and 23.

It would be desirable to develop derivatives of these compounds that do not similarly result in allergic reactions and/or which have improved useful lifetimes. Additionally beneficial properties include improved odor intensity and stability. The present invention provides such fragrances and flavorings.

### SUMMARY OF THE INVENTION

Improved fragrances and flavorings that have a longer useful shelf life than the parent compounds from which they are derived are disclosed. In particular, derivatives of aromachemicals that maintain the fragrance characteristics of the aromachemicals, while lowering the allergic properties, and which can possess a longer shelf-life than the parent compounds from which they are derived, are disclosed. Also disclosed are methods of making the derivatives, and articles of manufacture including the derivatives.

The patent compounds include one or more double bonds and the derivatives are prepared by replacing one or more double bonds in the parent molecule with a cyclopropyl group, or thiirane group, where the cyclopropyl group can be unsubstituted, or substituted with one or two lower alkyl, preferably methyl groups. The alkyl groups can optionally be substituted, for example, with electron donating, groups, electron with drawing groups, groups which increase the hydrophilicity or hydrophobocity, and the like.

Examples of suitable articles of manufacture include candles, air fresheners, perfumes, disinfectant compositions, hypochlorite (bleach) compositions, beverages such as beer and soda, denture cleanser tablets as described, for example, in U.S. Patent No.

5,571,519 and flavored orally-delivered products such as lozenges, randies, and the like.

### BRIEF DESCRIPTION OF THE DRAWINGS

Figures 1a-d are vibrational spectra of a cyclopropyl derivative of rose oxide (1a), linalool (1b), limonene (1c) and ionone (1d), whereby only linalool in part of the invention.

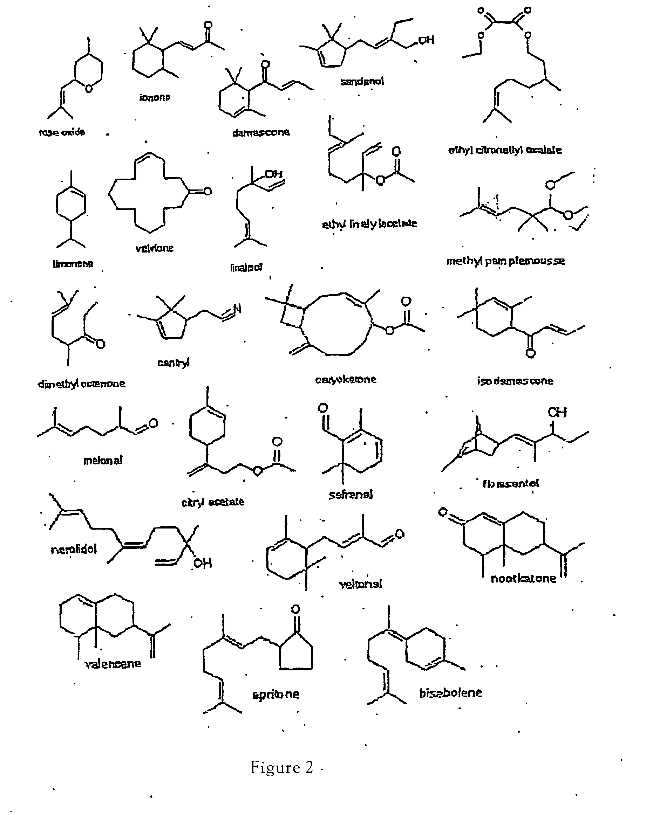

Figure 2 shows the structure linalool and a representative list of other aromachemicals that can be modified using the chemistry described herein, but which are not part of the invention.

### DETAILED DESCRIPTION OF THE INVENTION

Improved fragrances and flavorings that have a longer useful shelf life than the parent compounds from which they are derived are disclosed. The improvements can be in the form of greater intensity and/or greater chemical stability without change in odor character. If greater intensity is desired, then the odorant structure is modified in order to increase the intensity of the odor, such as by increasing zinc-binding ability, without significantly changing odor character. If greater stability is desired, then one or more structural features responsible for chemical instabitity can be altered as described herein without significantly changing odor character.

### 1. Isodonic molecules

The derivatives described herein are isodonic to the compounds from which they can be derived. By isodomic is meant "having essentially the same odor profile." However, while the compounds may have essentially the same odor profile, they have improved stability, odor intensity and/or other improved physical and/or chemical properties.

The compounds from which the derivatives can be derived are aromachemicals as defined in the claims. The derivatives can be prepared from the aromachemicals or the individual compounds, but need not be. That is, the compounds can be derived from synthetic strategies that do not involve using the aromachemicals, so long as the ultimate compound is a derivative of the specific odorant compounds as described herein. All that is required is that the compounds are isodonic with the "parent" compounds. Isodonic replacements (for example, enecyclopropane replacement, ene-thiirane replacement, and replacement, are described in more detail below.

The odorant intensity and/or stability of aromachemicals can be improved by replacing a common chemical feature with another designed to alter the chemistry while leaving the basic structure, and therefore the odor itself, virtually untouched. Examples of suitable chemical features that can be replaced are described in more detail below.

### Double bond Replacements and their Effect on Odor

Many aromachemicals include an isoprenyl unit and/or other C=C double bonds. In one embodiment, the carbon double bond is replaced with a thiocyclopropane ring. In this embodiment, the lone pair of electrons on sulfur binds readily to Zn, which increases the odor intensity without significantly altering the odor type.

One or more (if present) C=C double bonds in an essential oil can be replaced with cyclopropane, or thiirane (TH) moieties. The odor of the compounds remains substantially the same with this substitution, whereas the odorant intensity can be dramatically improved. One way to measure the odorant intensity is through zinc binding affinity. All three 3-membered rings described above can coordinate to a zinc ion.

Another advantage of replacing the C=C double bond with a thiirane is that this produces a molecule with a higher molecular weight. The greater molecular weight can lower the volatility of the molecule, thereby potentially changing a top not to a middle note, or a middle note to a drydown note.

The procedure described herein for improving the performance of an odorant is best illustrated with citral. It is immediately applicable to any other odorants possessing the same structural features, namely a C=C double bond. Citral can be cyclopropanated in one or both of the positions, corresponding to the two double bonds of the molecule. Including stereoisomers, there are thus five CP-variations on citral, shown below.

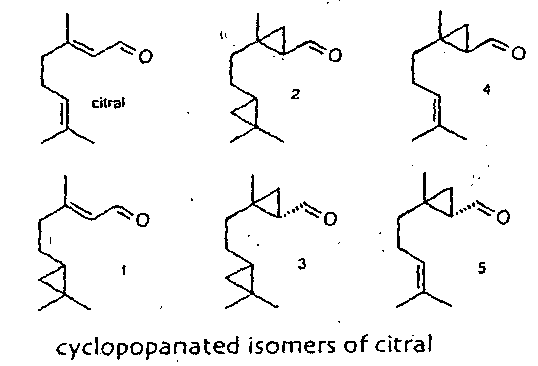

These citral derivatives are not part of the invention.

The cyclopropane rings can include a CH₂ moiety, or can be substituted with one or two methyl groups. The methyl or dimethyl analogues have a vibrational spectra that more closely matches citral than the unsubstituted cyclopropane derivatives, and has a sweeter smell than the unsubstituted cyclopropyl derivatives.

The derivatives described herein include derivatives in which one or both of the double bonds is replaced with a (unsubstituted, monoalkyl or dialkyl, where alkyl can be substituted or unsubstituted, and is preferably methyl) cyclopropyl group.

The synthesis of methyl, dimethyl or unsubstituted cyclopropane derivatives is well known to those of skill in the art, and involves, for example, bromoform reaction to form the dibromocyclopropane derivative, followed by stoichiometric reaction with methyl lithium.

These simple procedures yield derivatives with odor profiles close to the aromachemicals or individual "parent"' compounds themselves. Further, by replacing the double bonds, the derivatives often have greater potency and far greater acid and bleach stability since the unstable feature, namely the double bond, has been removed.

The same applies to thiirane (TH) rings, Not counting mixed C=C double bond replacements and stercoisomers, this generates 9 possible molecules from citral alone, all readily accessible in one or two step syntheses from citral by processes well known in the art, such as:
**Cyclopropanyl replacement:** Simmons-Smith cyclopropanation of the aldehyde or corresponding alcohol, followed by periodinane oxidation for the latter to give the aldehyde^{1 1} Vogel's textbook of practical organic chemistry 5th edition (1989) pp 1106-1108

Aromachemicals including a one or more a double bonds can be converted to a (unsubstituted, methyl or dimethyl) cyclopropyl, or thiirane derivative.

This method is immediately applicable to several other classes of odorants in order to increase their potencies. The following examples each denote a class of odorant, not a single molecule.

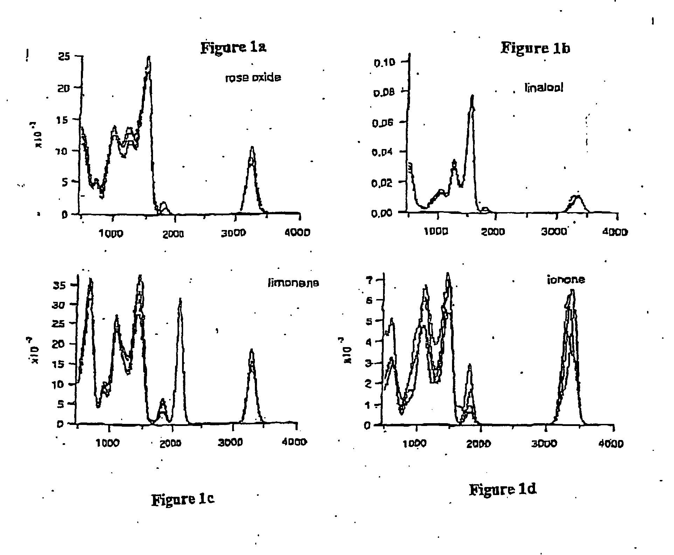

In each case the C=C double bond(s) can be substituted with CP, OX or TH to yield stronger odorants with similar odor profiles. Rose oxide is a floral, ionone a woodyviolets, damascone a fruity rose, sandanol a sandalwood, limonene a woody citrus, velvione a musk, linalool a floral-woody and ethyl citronellyl oxalate a musk. The graphs for four of these compounds with CP, OX and TH substitutions are shown in Figures 1a-d, where Figure 1a shows spectra of rose oxide, Figure 1b shows spectra for linalool, Figure 1c shows spectra for limonene and Figure 1d shows spectra for ionone. In each case, the CP, OX and TH substitution has only a minor effect on spectrum and therefore on odor character. Only the compounds as defined in the claims are part of the invention.

### II. Aromachemicals That Can be Modified Using the Chemistry Described Herein

The technology described herein has particular application to aromachsmicals Examples of aromachemicals that can be modified using the chemistry described herein are listed below, and also provided in Figure 3, whereby only linalool is part of the invention.

There are several aromachemicals that include olefinic groups that can be derivatized by forming an ene-cycloproane, and/or ene-thiirane replacement. These include citronellal and linalool. Thus compounds that can be used in the present invention are as defined in claim 1. In a preferred aspect the compounds used in the invention comprise at least one C(R)₂ group, wherein at least one R in the C(R)₂ group is methyl. Citronellal and linalool can be modified as described herein, wherein the double bonds can be converted to (unsubstituted, methyl or dimethyl) cyclopropyl derivatives without significantly altering the odor profile.

Thus, compounds that can be used in the present invention are defined in claim 1. In a preferred aspect the compounds used in the invention comprise at least one C(R)₂ group, wherein at least one 12 in the C(R)₂ group is methyl.

### III. Articles of Manufacture Including the Essential Oil Derivatives

The derivatives described herein can be included in virtually any article of manufacture that can include the aromachemicals or other "parent compounds" from which they are derived. Examples include bleach, detergents, flavorings and fragrances, beverages. including alcoholic beverages, and the like. The derivatives can be used in applications like soaps, shampoos, body deodorants and antiperspirants, solid or liquid detergents for treating textiles, fabric softeners, detergent compositions and/or all-purpose cleaners for cleaning dishes or various surfaces, for both household and industrial use. Of course, the use of the compounds is not limited to the above-mentioned products, as they be used in other current uses in perfumery, namely the perfuming of soaps and shower gels, hygiene or hair-care products, as well as of body deodorants, air fresheners and cosmetic preparations, and even in fine perfumery, namely in perfumes and colognes. These uses are described in more detail below.

### Perfume Compositions

The compounds can be used as perfuming ingredients, as single compounds or as mixture thereof, preferably at a range of at least about 30% by weight of the perfume composition, more preferably at a range of at least about 60% by weight of the composition. The compounds can even be used in their pure state or as mixtures, without added components. The olfactive characteristics of the individual compounds are also present in mixtures thereof, and mixtures of these compounds can be used as perfuming ingredients. This may be particularly advantageous where separation and/or purification steps can be avoided by using compound mixtures.

In all cited applications, the derivatives can be used alone or in admixture with other perfuming ingredients, solvents or adjuvants of current use in the art. The nature and the variety of these co-ingredients do not require a more detailed description here, which, moreover, would not be exhaustive, and the person skilled in the art will be able to choose the latter through its general knowledge and as a function of the nature of the product to be perfumed and of the desired olfactive effect.

These perfuming ingredients typically belong to chemical classes as varied as alcohols, aldehydes, ketones, esters, ethers, acetates, nitrites, terpene hydrocarbons, sulfur and nitrogen-containing heterocyclic compounds, as well as aromachemicals of natural or synthetic origin. A large number of these ingredients described in reference textbooks such as the book of S. Arctander, Perfume and Flavor Chemicals, 1969, Montclair, N.J., USA, the contents of which are hereby incorporated by reference in its entirety, or its more recent versions, or in other works of similar nature.

The proportions in which the derivatives can be incorporated in the various products vary within a large range of values. These values depend on the nature of the article or product that one desires to perfume and the odor effect searched for, as well as on the nature of the co-ingredients in a given composition when the compounds are used in admixture with perfuming co-ingredients, solvents or adjuvants of current use in the art.

As an example, the derivatives are typically present at concentrations between about 0.1 and about 10%, or even more, by weight of these compounds relative to the weight of the perfuming composition in which they are incorporated. Far lower concentrations than those mentioned above can be used when the compounds are directly applied for perfuming the various consumer products cited beforehand.

The compounds are relatively stable in typically aggressive media for perfumes. Accordingly, they can be used in detergents containing bleaching agents and activators such as, for example, tetraacetylethylenediamine (TAED), hypohalites, in particular hypochlorite, peroxygenated bleaching agents such as, for example, perborates, etc. The compounds can also be used in body deodorants and antiperspirants, for example, those containing aluminum salts. These embodiments are described in more detail below.

### Conventional Detergent Ingredients

In addition to the derivatives described herein, the compositions herein include a detersive surfactant and optionally, one or more additional detergent ingredients, including materials for assisting or enhancing cleaning performance, treatment of the substrate to be cleaned, or to modify the aesthetics of the detergent composition (e.g., perfumes, colorants, dyes, etc.). The following are illustrative examples of detersive surfactants and other detergent ingredients.

Detersive Surfactants Non-limiting examples of synthetic detersive surfactants useful herein typically at levels from about 0.5% to about 90%, by weight, include the conventional C₁₁₋₁₈ alkyl benzene sulfonates ("LAS") and primary, branch-chain and random C₁₀₋₂₀ alkyl sulfates ("AS"), the C₁₀₋₁₈ secondary (2,3) alkyl sulfates of the formula CH₃ (CH₂)ₓ (CH(CH₃)OSO₃⁻ M⁺) and CH₃(CH₂)_{y} (CH(CH₂CH₂)OSO₃⁻ M⁺) wherein x and y are integers and wherein each of x and (y+1) is least about 7, preferably at least about 9, and M is a water-solubilizing cation, especially sodium, unsaturated sulfates such as oleyl sulfate, the C₁₀₋₁₈ alkyl alkoxy sulfates ("AEx S"; especially EO 1-7 ethoxy sulfates), C₁₀₋₁₈ alkyl alkoxy carboxylates (especially the EO 1-5 ethoxycarboxylates), the C₁₀₋₁₈ glycerol ethers, the C₁₀₋₁₈ alkyl polyglycosides and their corresponding sulfated polyglycosides, and C₁₂₋₁₈ alpha-sulfonated fatty acid esters. If desired, the conventional nonionic and amphoteric surfactants such as the C₁₂₋₁₈ alkyl ethoxylates ("AE") including the so-called narrow peaked alkyl ethoxylates and C₆₋₁₂ alkyl phenol alkoxylates (especially ethoxylates and mixed ethoxy/propoxylates), C₁₂₋₁₈ betaines and sulfobetaines ("sultaines"), C₁₀₋₁₈ amine oxides, and the like, can also be included in the overall compositions. The C₁₀₋₁₈ N-alkyl polyhydroxy fatty acid amides can also be used. Typical examples include the C₁₂₋₁₈ N-methylglucamides. See WO 9,206,154. Other sugar-derived surfactants include the N-alkoxy polyhydroxy fatty acid amides, such as C₁₀₋₁₈ N-(3-methoxypropyl) glucamide. The N-propyl through N-hexyl C₁₂₋₁₈ glucamides can be used for low sudsing. Q₁₀₋₂₀ conventional soaps may also be used, however synthetic detergents are preferred. If high sudsing is desired, the branched-chain C₁₀₋₁₆ soaps may be used. Mixtures of anionic and nonionic surfactants are especially useful. Other conventional useful surfactants are listed in standard texts. See also U.S. Pat. No. 3,664,961, Norris, issued May 23, 1972.

Preferred compositions incorporating only synthetic detergents have a detergent level of from about 0.5% to 50%. Compositions containing soap preferably comprise from about 10% to about 90% soap.

Although the detergent compositions herein can consist of only detersive surfactant and pro-fragrance, the said compositions preferably contain other ingredients commonly used in detergent products.

### Builders

Detergent builders can optionally be included in the compositions herein to assist in controlling mineral hardness. Inorganic as well as organic builders can be used. Builders are typically used in fabric laundering compositions to assist in the removal of particulate soils.

The level of builder can vary widely depending upon the end use of the composition and its desired physical form. When present, the compositions will typically comprise at least about 1% builder. Liquid formulations typically comprise from about 5% to about 50%, more typically about 5% to about 30%, by weight, of detergent builder. Granular formulations typically comprise from about 10% to about 80%, more typically from about 15% to about 50% by weight, of the detergent builder. Lower or higher levels of builder, however, are not meant to be excluded.

Inorganic or detergent builders include, but are not limited to phosphate builders such as, the alkali metal, ammonium and allanolammonium salts of polyphosphates (exemplified by the tripolyphosphates, pyrophosphates, and glassy polymeric meta-phosphates), phosphonates, and phytic acid, and non-phosphorous builders such as silicates, carbonates (including bicarbonates and sesquicarbonates), sulphates, and aluminosilicates. Non-phosphate builders are required in some locales.

Organic builders suitable for use herein include polycarboxylate builders such as disclosed in U.S. Pat. No. 3,308,067, Diehl issued Mar. 7, 1967; U.S. Pat. No. 4,144,226, Crutchfield issued Mar. 13, 1979 and U.S. Pat. No. 4,246,495, Crutchfield, issued Mar. 27, 1979.

### Soil Release Agents

Soil Release agents are desirably used in laundry detergents of the instant invention. Suitable soil release agents include those of U.S. Pat. No. 4,968,451, Nov. 6, 1990 to J. J. Scheibel and E. P. Gosselink: such ester oligomers can be prepared by (a) ethoxylating allyl alcohol, (b) reacting the product of (a) with dimethyl terephthalate ("DMT") and 1,2-propylene glycol ("PG") in a two-stage transesterification/oligomerization procedure and (c) reacting the product of (b) with sodium metabisulfite in water; the nonionic end-capped 1,2-propylene/polyoxyethylene terephthalate polyesters of U.S. Pat. No. 4,711,730, Dec. 8, 1987 to Gosselink et al, for example those produced by transesterification/oligomerization of poly(ethyleneglycol) methyl ether, DMT, PG and poly(ethyleneglycol) ("PEG"); the partly- and fully-anionic-end-apped oligomeric esters of U.S. Pat. No. 4,721,580, Jan. 26, 1988 to Gosselink, such as oligomers from ethylene glycol ("EG"), PG, DMT and Na-3,6-dioxa-8-hydroxyoctanesulfonate; the nonionic-capped block polyester oligomeric compounds of U.S. Pat. No. 4,702,857, Oct. 27, 1987 to Gosselink, for example produced from DMT, Mecapped PEG and EG and/or PG, or a combination of DMT, EG and/or PG, Macapped PEG and Na-dimethyl-5-sulfoisophthalate; and the anionic, especially sulfoaroyl, end-capped terephthalate esters of U.S. Pat. No. 4,877,896, Oct. 31, 1989 to Maldonado, Gosselink et al, the latter being typical of SRA's useful in both laundry and fabric conditioning products, an example being an ester composition made from m-sulfobenzoic acid monosodium salt, PG and DMT optionally but preferably further comprising added PEG, e.g., PEG 3400. Another preferred soil release agent is a sulfonated end-capped type described in U.S. Pat. No. 5,415,807.

### Other Optional Ingredients

The compositions herein can contain other ingredients such as enzymes, bleaches, fabric softening agents, dye transfer inhibitors, suds suppressors, and chelating agents, all well known within the art.

For purposes of defining detergent compositions of the present invention, the pH of the detergent composition is that which is measured at 1% concentration of the detergent composition in distilled-water at 20C. The detergent compositions herein have a pH of from about 7.1 to about 13, more typically from about 7.5 to about 9.5 for liquid detergents and from about 8 to about 12 for granular detergents.

### Formulation with Detergents With or Without Conventional Perfumery Materials

While the derivatives described herein can be used alone and simply mixed with essential detergent ingredient, most notably surfactant, they can also be desirably combined into three-part formulations which combine (a) a non-fragranced detergent base comprising one or more synthetic detergents and (b) one or more of the derivatives described herein. In one embodiment, both aldehydes and acetals are present, such that the aldehydes provide desirable in-package and in-use (wash-time) fragrance, while the acetals provide a long-term fragrance to the laundered textile fabrics.

In formulating the present detergents, the fully-formulated fragrance can be prepared using numerous known odorant ingredients of natural or synthetic origin. The range of the natural raw substances can embrace not only readily-volatile, but also moderately-volatile and slightly-volatile components and that of the synthetics can include representatives from practically all classes of fragrant substances, as will be evident from the following illustrative compilation: natural products, such as tree moss absolute, basil oil, citrus fruit oils (such as bergamot oil, mandarin oil, etc.), mastix absolute, myrtle oil, palmarosa oil, patchouli oil, petitgrain oil Paraguay, wormwood oil, alcohols, such as farnesol, geraniol, linalool, nerol, phenylethyl alcohol, rhodinol, cinnamic alcohol, aldehydes, such as citral, Helional.TM., alpha-hexyl-cinnamaldehyde, hydroxycitronellal, Lilial.TM. (p-tert.butyl-alpha-methyldihydrocinnamaldehyde), methylaonylacetaldehyde, ketones, such as allylionone, alpha-ionone, beta-ionone, isoraldein (isomethyl-alpha-ionone), methylionone, esters, such as allyl phenoxyacetate, benzyl salicylate, cinnamyl propionate, citronellyl acetate, citronellyl ethoxolate, decyl acetate, dimethylbenzylcarbinyl acetate, dimethylbenzylcarbinyl butyrate, ethyl acetoacetate, ethyl acetylacetate, hexenyl isobutyrate, linalyl acetate, methyl dihydrojasmonate, styrallyl acetate, vetiveryl acetate, etc., lactones, such as gamma-undecalactone, various components often used in perfumery, such as musk ketone, indole, p-menthane-8-thiol-3-one, and methyl-eugenol. Likewise, any conventional fragrant acetal or ketal known in the art can be added to the present composition as an optional component of the conventionally formulated perfume (c). Such conventional fragrant acetals and ketals include the well-known methyl and ethyl acetals and ketals, as well as acetals or ketals based on benzaldehyde, those comprising phenylethyl moieties, or more recently developed specialties such as those described in a United States Patent entitled "Acetals and Ketals of Oxo-Tetralins and Oxo-Indanes, see U.S. Pat. No. 5,084,440, issued Jan. 28, 1992, assigned to Givaudan Corp. Of course, other recent synthetic specialties can be included in the perfume compositions for fully-formulated detergents. These include the enol ethers of alkylsubstituted oxo-tetralins and oxo-indanes as described in U.S. Pat. No. 5,332,725, Jul. 26, 1994, assigned to Givaudan; or Schiff Bases as described in U.S. Pat. No. 5,264,615, Dec. 9, 1991, assigned to Givaudan. It is preferred that the pro-fragrant material be added separately from the conventional fragrances to the detergent compositions of the invention.

### Formulation with other Special-Purpose Fragrance Delivering Compounds

Detergents including the derivatives described herein may further, optionally, if desired, contain other known compounds having the capability to enhance substantivity of a fragrance. Such compounds include, but are not limited to, the aluminium alkoxides such as isobutylaluminium diferanylate as disclosed in U.S. Pat. No. 4,055,634; or the known titanate and zirconate esters or oligoesters of fragrant materials such as those disclosed in U.S. Pat. No. 3,947,574, and U.S. Pat. No. 3,779,932, the contents of each of which are hereby incorporated by reference in their entirey. When using such organoaluminum, organotitanium or organozinc derivatives, they may be incorporated into the present formulations at their art-known levels.

### Beverage Compositions

The improved flavorings described herein can be incorporated into beverages and impart various flavorings to the beverages. The preferred flavor is lemon, but additional flavors include rose, cinnamon, lime, and the like. The beverage composition can be a cola beverage composition, and can also be coffee, tea, dairy beverage, fruit juice drink, orange drink, lemon-lime drink, beer, malt beverages, or other flavored beverage. The beverages can be in liquid or powdered form.

The beverage compositions can also include one or more flavoring agents; artificial colorants; vitamin additives; preservatives; caffeine additives; water; acidulants; thickeners; buffering agents; emulsifiers; and or fruit juice concentrates.

Artificial colorants which may be used include caramel color, yellow 6 and yellow 5. Useful vitamin additives include vitamin B2, vitamin B6, vitamin B12, vitamin C (ascorbic acid), niacin, pantothenic acid, biotin and folic acid. Suitable preservatives include sodium or potassium benzoate. Salts which may be used include sodium, potassium and magnesium chloride. Exemplary emulsifiers are gum arabic and purity gum, and a useful thickener is pectin. Suitable acidulants include citric, phosphoric and malic acid, and potential buffering agents include sodium and potassium citrate.

In one embodiment, the beverage is a carbonated cola beverage. The pH is generally about 2.8 and the following ingredients can be used to make the syrup for these compositions: Flavor Concentrate, including one or more of the derivatives described herein (22.22 ml), 80% Phosphoric Acid (5.55 g), Citric Acid (0.267 g), Caffeine (1.24 g), artificial sweetener, sugar or corn syrup (to taste, depending on the actual sweetener) and Potassium Citrate (4.07 g). The beverage composition can be prepared, for example, by mixing the foregoing syrup with carbonated water in a proportion of 50 ml syrup to 250 ml of carbonated water.

In another embodiment, the beverage is a beer or malt beverage. Preferred flavorings for beer and malt beverages include lemon, lime and lemon-lime. Advantageously, the flavorings include citral derivatives in which one of both of the double bonds are replaced with a cyclopropane group, where the cyclopropane groups can, independently, be unsubstituted, or include one or two alkyl or substituted alkyl groups, preferably methyl groups. The amount of flavoring can be adjusted according to taste.

### Orally-Delivered Products

Flavored food and pharmaceutical compositions including one or more of the derivatives described herein can also be prepared. The derivatives can be incorporated into conventional foodstuffs using techniques well known to those of skill in the art. Alternatively, the derivatives can be incorporated within polymeric particles, which can, in turn, be dispersed within and/or over a surface of an orally-deliverable matrix material, which is usually a solid or semi-solid substrate. When used in chewable compositions, the derivatives can be released into the orally-deliverable polymeric matrix material as the composition is chewed and held in the mouth, thus prolonging the flavor of the composition. In the case of dried powders and mixes, the flavor can be made available as the product is consumed or be released into the matrix material as the composition is further processed. When two flavors are combined with the polymeric particles, the relative amounts of the additives can be selected to provide simultaneous release and exhaustion of the compounds.

In one embodiment, the flavored composition includes an orally-deliverable matrix material; a plurality of water insoluble polymeric particles dispersed in the orally-deliverable matrix material, where the polymeric particles individually define networks of internal pores and are non-degradable in the digestive tract; and one or more derivatives as described herein entrapped within the internal pore networks. The derivatives are released as the matrix is chewed, dissolved in the mouth, or undergoes further processing selected from the group consisting of liquid addition, dry blending, stirring, mixing, heating, baking, and cooking. The orally-deliverable matrix material can be selected from the group consisting of gums, latex materials, crystallized sugars, amorphous sugars, fondants, nougats, jams, jellies, pastes, powders, dry blends, dehydrated food mixes, baked goods, batters, doughs, tablets, and lozenges.

### Chewing Gum

A flavorless gum base can be combined with a citral or other suitable derivative as described herein to a desired flavor concentration. Typically, a blade mixer is heated to about 110F, the gum base is preheated so that it is softened, and the gum base is then added to the mixer and allowed to mix for approximately 30 seconds. The flavored derivative is then added to the mixer and mixed for a suitable amount of time. The gum can be then removed from the mixer and rolled to stick thickness on waxed paper while warm.

### Time Release Formulations

In one embodiment, the derivatives described herein are incorporated into a system which can release a fragrance in a controlled manner. These include substrates such as air fresheners, laundry detergents, fabric softeners, deodorants, lotions, and other household items. The fragrances are generally one or more derivatives of aromachemicals as described herein, each present in different quantities. U.S. Pat. No. 4,587,129, the contents of which are hereby incorporated by reference, in their entirety, describes a method for preparing gel articles which contain up to 90% by weight of fragrance or perfume oils. The gels are prepared from a polymer having a hydroxy (lower alkoxy) 2-alkeneoate, a hydroxy (lower alkoxy) lower alkyl 2-alkeneoate, or a hydroxy poly (lower alkoxy) lower alkyl 2-alkeneoate and a polyethylenically unsaturated crosslinking agent. These materials have continuous slow release properties, i.e., they release the fragrance component continuously over a long period of time. Advantageously, all or a portion of those derivatives that include an aldehyde group can be modified to include an acetal group, which can cause the formulations to release fragrance over a period of time as the acetal hydrolyzes to form the aldehyde compound.

## Claims

1. The use, as a flavouring and/or fragrance, of a compound obtainable by a process which comprises replacing at least one carbon-carbon double bond in a starting material selected from citronellol and linalool by a three-membered ring, wherein the three membered ring includes the two carbons of the double bond and a sulfur atom or a C(R)₂ group wherein each R is independently H, C₁₋₅ alkyl or C₁₋₅ substituted alkyl, and wherein the substituents are selected from the group consisting of halo, hydroxy, thiol, thioether, amine, carboxylic acid, ester, nitro, cyano, sulfonic acid, urea and thiourea.

2. Use according to claim 1, wherein the compound comprises at least one C(R)₂ group, wherein at least one R in the C(R)₂ group is methyl.

3. Use according to claim 1, wherein the compound comprises at least one three membered ring comprising a sulfur atom.

4. The use of an unsubstituted-, methyl- or dimethyl-cyclopropyl derivative of citronellol or linalool as a flavouring and/or a fragrance.

5. Use according to any one of claims 1 to 4 in a composition comprising a perfuming ingredient, a solvent, or an adjuvant of current use in the art of perfumery.

6. Use according to claim 5, wherein the compound defined in claims 1 to 4 is present in an amount of at least 30 percent by weight.

7. Use according to claim 6, wherein the compound defined in claims 1 to 4 is present in an amount of at least 60 percent by weight.

8. A perfumed article comprising a compound obtainable by a process which comprises replacing at least one carbon-carbon double bond in a starting material selected from citronellol and linalool by a three-membered ring, wherein the three membered ring includes the two carbons of the double bond and a sulfur atom or a C(R)₂ group wherein each R is independently H, C₁₋₅ alkyl or C₁₋₅ substituted alkyl, and wherein the substituents are selected from the group consisting of halo, hydroxy, thiol, thioether, amine, carboxylic acid, ester, nitro, cyano, sulfonic acid, urea and thiourea.

9. An article according to claim 8 wherein the compound comprises at least one C(R)₂ group, wherein at least one R in the C(R)₂ group is methyl.

10. An article according to claim 8, wherein the compound comprises at least one three membered ring comprising a sulfur atom.

11. A perfumed article comprising an unsubstituted-, methyl- or dimethyl-cyclopropyl derivative of citronellol or linalool.

12. An article according to any one of claims 8 to 11 comprising a perfuming ingredient, a solvent, or an adjuvant of current use in the art of perfumery.

13. An article according to claim 12, wherein the compound as defined in claims 8 to 11 is present in an amount of at least 30 percent by weight.

14. An article according to claim 13, wherein the compound as defined in claims 8 to 11 is present in an amount of at least 60 percent by weight.

15. A perfumed article according to any one of claims 8 to 11, in the form of a perfume or cologne, a soap, a bath or shower gel, a shampoo or other hair care product, a cosmetic preparation, a body deodorant or antiperspirant, an air freshener, a fabric detergent or softener or an all-purpose household cleaner.

16. A body deodorant or antiperspirant according to claim 15.

17. A detergent comprising a compound obtainable by a process which comprises replacing at least one carbon-carbon double bond in a starting material selected from citronellol and linalool by a three-membered ring, wherein the three membered ring includes the two carbons of the double bond and a sulfur atom or a C(R)₂ group wherein each R is independently H, C₁₋₅ alkyl or C₁₋₅ substituted alkyl, and wherein the substituents are selected from the group consisting of halo, hydroxy, thiol, thioether, amine, carboxylic acid, ester, nitro, cyano, sulfonic acid, urea and thiourea.

18. A detergent according to claim 17 wherein the compound comprises at least one C(R)₂ group, wherein at least one R in the C(R)₂ group is methyl.

19. A detergent according to claim 17, wherein the compound comprises at least one three membered ring comprising a sulfur atom.

20. A detergent comprising an unsubstituted-, methyl- or dimethyl-cyclopropyl derivative of citronellol or linalool.

21. A bleach composition comprising a compound obtainable by a process which comprises replacing at least one carbon-carbon double bond in a starting material selected from citronellol and linalool by a three-membered ring, wherein the three membered ring includes the two carbons of the double bond and a sulfur atom or a C(R)₂ group wherein each R is independently H, C₁₋₅ alkyl or C₁₋₅ substituted alkyl, and wherein the substituents are selected from the group consisting of halo, hydroxy, thiol, thioether, amine, carboxylic acid, ester, nitro, cyano, sulfonic acid, urea and thiourea.

22. A bleach composition according to claim 21 wherein the compound comprises at least one C(R)₂ group, wherein at least one R in the C(R)₂ group is methyl.

23. A bleach composition according to claim 21, wherein the compound comprises at least one three membered ring comprising a sulfur atom.

24. A bleach composition comprising an unsubstituted-, methyl- or dimethyl-cyclopropyl derivative of citronellol or linalool.

25. A beverage comprising a compound obtainable by a process which comprises replacing at least one carbon-carbon double bond in a starting material selected from citronellol and linalool by a three-membered ring, wherein the three membered ring includes the two carbons of the double bond and a sulfur atom or a C(R)₂ group wherein each R is independently H, C₁₋₅ alkyl or C₁₋₅ substituted alkyl, and wherein the substituents are selected from the group consisting of halo, hydroxy, thiol, thioether, amine, carboxylic acid, ester, nitro, cyano, sulfonic acid, urea and thiourea.

26. A beverage according to claim 25 wherein the compound comprises at least one C(R)₂ group, wherein at least one R in the C(R)₂ group is methyl.

27. A beverage according to claim 25, wherein the compound comprises at least one three membered ring comprising a sulfur atom.

28. A beverage comprising an unsubstituted-, methyl- or dimethyl-cyclopropyl derivative of citronellol or linalool.

29. A beverage according to any one of claims 25 to 28, wherein the beverage is selected from the group consisting of beer, malt liquor, lemonade and cola.

30. A flavoured orally-delivered product comprising a compound obtainable by a process which comprises replacing at least one carbon-carbon double bond in a starting material selected from citronellol and linalool by a three-membered ring, wherein the three membered ring includes the two carbons of the double bond and a sulfur atom or a C(R)₂ group wherein each R is independently H, C₁₋₅ alkyl or C₁₋₅ substituted alkyl, and wherein the substituents are selected from the group consisting of halo, hydroxy, thiol, thioether, amine, carboxylic acid, ester, nitro, cyano, sulfonic acid, urea and thiourea.

31. A flavoured orally-delivered product according to claim 30 wherein the compound comprises at least one C(R)₂ group, wherein at least one R in the C(R)₂ group is methyl.

32. A flavoured orally-delivered product according to claim 30, wherein the compound comprises at least one three membered ring comprising a sulfur atom.

33. A flavoured orally-delivered product comprising an unsubstituted-, methyl- or dimethyl-cyclopropyl derivative of citronellol or linalool.

34. A method to improve, enhance, or modify the odour of a perfuming composition or a perfumed article comprising adding at least one compound obtainable by a process which comprises replacing at least one carbon-carbon double bond in a starting material selected from citronellol and linalool by a three-membered ring, wherein the three membered ring includes the two carbons of the double bond and a sulfur atom or a C(R)₂ group wherein each R is independently H, C₁₋₅ alkyl or C₁₋₅ substituted alkyl, and wherein the substituents are selected from the group consisting of halo, hydroxy, thiol, thioether, amine, carboxylic acid, ester, nitro, cyano, sulfonic acid, urea and thiourea to the composition or the article.

35. A method according to claim 34 wherein the compound comprises at least one C(R)₂ group, wherein at least one R in the C(R)₂ group is methyl.

36. A method according to claim 34, wherein the compound comprises at least one three membered ring comprising a sulfur atom.

37. A method to improve, enhance, or modify the odour of a perfuming composition or a perfumed article comprising adding at least one of an unsubstituted-, methyl- or dimethyl-cyclopropyl derivative of citronellol or linalool to the composition or the article.

38. A method according to any one of claims 34 to 37, wherein the at least one compound is present in admixture with a perfuming ingredient, a solvent, or an adjuvant of current use in the art of perfumery.

39. A method according to any one of claims 34 to 37, wherein the at least one compound is present in an amount of at least 30 percent by weight.

## Patentansprüche

1. Verwendung einer Verbindung, die durch ein Verfahren erhältlich ist, das das Ersetzen von mindestens einer Kohlenstoff-Kohlenstoff-Doppelbindung in einem Ausgangsmaterial, das aus Citronellol und Linalool ausgewählt ist, durch einen dreigliedrigen Ring umfasst, wobei der dreigliedrige Ring die zwei Kohlenstoffe der Doppelbindung und ein Schwefelatom oder eine C(R)₂₋Gruppe, worin jedes R unabhängig voneinander für H, C₁₋₅-Alkyl oder substituiertes C₁₋₅-Alkyl steht und wobei die Substituenten aus der Gruppe von Halogen, Hydroxy, Thiol, Thioether, Amin, Carbonsäure, Ester, Nitro, Cyano, Sulfonsäure, Harnstoff und Thioharnstoff ausgewählt sind, umfasst, als Aromastoff und/oder Duftstoff.

2. Verwendung nach Anspruch 1, wobei die Verbindung mindestens eine C(R)₂-Gruppe umfasst, wobei mindestens ein R in der C(R)₂-Gruppe Methyl ist.

3. Verwendung nach Anspruch 1, wobei die Verbindung mindestens einen, ein Schwefelatom umfassenden dreigliedrigen Ring umfasst.

4. Verwendung eines unsubstituierten, Methyl- oder Dimethyl-cyclopropylderivats von Citronellol oder Linalool als Aroma- und/oder Duftstoff.

5. Verwendung nach einem der Ansprüche 1 bis 4 in einer Zusammensetzung, die einen Parfümbestandteil, ein Lösemittel oder ein derzeit auf dem Gebiet der Parfümeriewaren verwendetes Adjuvans umfasst.

6. Verwendung nach Anspruch 5, wobei die in den Ansprüchen 1 bis 4 definierte Verbindung in einer Menge von mindestens 30 Gew.-% vorhanden ist.

7. Verwendung nach Anspruch 6, wobei die in den Ansprüchen 1 bis 4 definierte Verbindung in einer Menge von mindestens 60 Gew.-% vorhanden ist.

8. Parfümierter Gegenstand, der eine Verbindung umfasst, die durch ein Verfahren erhältlich ist, das das Ersetzen von mindestens einer Kohlenstoff-Kohlenstoff-Doppelbindung in einem Ausgangsmaterial, das aus Citronellol und Linalool ausgewählt ist, durch einen dreigliedrigen Ring umfasst, wobei der dreigliedrige Ring die zwei Kohlenstoffe der Doppelbindung und ein Schwefelatom oder eine C(R)₂-Gruppe, worin jedes R unabhängig voneinander für H, C₁₋₅-Alkyl oder substituiertes C₁₋₅-Alkyl steht und wobei die Substituenten aus der Gruppe von Halogen, Hydroxy, Thiol, Thioether, Amin, Carbonsäure, Ester, Nitro, Cyano, Sulfonsäure, Harnstoff und Thioharnstoff ausgewählt sind, umfasst.

9. Gegenstand nach Anspruch 8, wobei die Verbindung mindestens eine C(R)₂-Gruppe umfasst, wobei mindestens ein R in der C(R)₂-Gruppe Methyl ist.

10. Gegenstand nach Anspruch 8, wobei die Verbindung mindestens einen, ein Schwefelatom umfassenden dreigliedrigen Ring umfasst.

11. Parfümierter Gegenstand, der ein unsubstituiertes, Methyl- oder Dimethyl-cyclopropylderivat von Citronellol oder Linalool umfasst.

12. Gegenstand nach einem der Ansprüche 8 bis 11, der einen Parfümbestandteil, ein Lösemittel oder ein derzeit auf dem Gebiet der Parfümeriewaren verwendetes Adjuvans umfasst.

13. Gegenstand nach Anspruch 12, wobei die in den Ansprüchen 8 bis 11 definierte Verbindung in einer Menge von mindestens 30 Gew.-% vorhanden ist.

14. Gegenstand nach Anspruch 13, wobei die in den Ansprüchen 8 bis 11 definierte Verbindung in einer Menge von mindestens 60 Gew.-% vorhanden ist.

15. Parfümierter Gegenstand gemäß einem der Ansprüche 8 bis 11 in der Form eines Parfüms oder Eau de Cologne, einer Seife, eines Bade- oder Duschgels, eines Shampoos oder anderen Haarpflegeprodukts, einer Kosmetikzubereitung, eines Körperdeodorants oder Antitranspirationsmittels, eines Lüftverbesserers, eines Gewebereinigungsmittels oder Weichspülers oder eines Allzweckhaushaltsreinigers.

16. Körperdeodorant oder Antitranspirationsmittel nach Anspruch 15.

17. Reinigungsmittel, das eine Verbindung umfasst, die durch ein Verfahren erhältlich ist, das das Ersetzen von mindestens einer Kohlenstoff-Kohlenstoff-Doppelbindung in einem Ausgangsmaterial, das aus Citronellol und Linalool ausgewählt ist, durch einen dreigliedrigen Ring umfasst, wobei der dreigliedrige Ring die zwei Kohlenstoffe der Doppelbindung und ein Schwefelatom oder eine C(R)₂-Gruppe, worin jedes R unabhängig voneinander für H, C₁₋₅-Alkyl oder substituiertes C₁₋₅₋Alkyl steht und wobei die Substituenten aus der Gruppe von Halogen, Hydroxy, Thiol, Thioether, Amin, Carbonsäure, Ester, Nitro, Cyano, Sulfonsäure, Harnstoff und Thioharnstoff ausgewählt sind, umfasst.

18. Reinigungsmittel nach Anspruch 17, wobei die Verbindung mindestens eine C(R)₂-Gruppe umfasst, wobei mindestens ein R in der C(R)₂-Gruppe Methyl ist.

19. Reinigungsmittel nach Anspruch 17, wobei die Verbindung mindestens einen, ein Schwefelatom umfassenden dreigliedrigen Ring umfasst.

20. Reinigungsmittel, das ein unsubstituiertes, Methyl- oder Dimethyl-cyclopropylderivat von Citronellol oder Linalool umfasst.

21. Bleichzusammensetzung, die eine Verbindung umfasst, die durch ein Verfahren erhältlich ist, das das Ersetzen von mindestens einer Kohlenstoff-Kohlenstoff-Doppelbindung in einem Ausgangsmaterial, das aus Citronellol und Linalool ausgewählt ist, durch einen dreigliedrigen Ring umfasst, wobei der dreigliedrige Ring die zwei Kohlenstoffe der Doppelbindung und ein Schwefelatom oder eine C(R)₂-Gruppe, worin jedes R unabhängig voneinander für H, C₁₋₅-Alkyl oder substituiertes C₁₋₅-Alkyl steht und wobei die Substituenten aus der Gruppe von Halogen, Hydroxy, Thiol, Thioether, Amin, Carbonsäure, Ester, Nitro, Cyano, Sulfonsäure, Harnstoff und Thioharnstoff ausgewählt sind, umfasst.

22. Bleichzusammensetzung nach Anspruch 21, wobei die Verbindung mindestens eine C(R)₂-Gruppe umfasst, wobei mindestens ein R in der C(R)₂-Gruppe Methyl ist.

23. Bleichzusammensetzung nach Anspruch 21, wobei die Verbindung mindestens einen, ein Schwefelatom umfassenden dreigliedrigen Ring umfasst.

24. Bleichzusammensetzung, die ein unsubstituiertes, Methyl- oder Dimethyl-cyclopropylderivat von Citronellol oder Linalool umfasst.

25. Getränk, das eine Verbindung umfasst, die durch ein Verfahren erhältlich ist, das das Ersetzen von mindestens einer Kohlenstoff-Kohlenstoff-Doppelbindung in einem Ausgangsmaterial, das aus Citronellol und Linalool ausgewählt ist, durch einen dreigliedrigen Ring umfasst, wobei der dreigliedrige Ring die zwei Kohlenstoffe der Doppelbindung und ein Schwefelatom oder eine C(R)₂-Gruppe, worin jedes R unabhängig voneinander für H, C₁₋₅-Alkyl oder substituiertes C₁₋₅-Alkyl steht und wobei die Substituenten aus der Gruppe von Halogen, Hydroxy, Thiol, Thioether, Amin, Carbonsäure, Ester, Nitro, Cyano, Sulfonsäure, Harnstoff und Thioharnstoff ausgewählt sind, umfasst.

26. Getränk nach Anspruch 25, wobei die Verbindung mindestens eine C(R)₂-Gruppe umfasst, wobei mindestens ein R in der C(R)₂-Gruppe Methyl ist.

27. Getränk nach Anspruch 25, wobei die Verbindung mindestens einen, ein Schwefelatom umfassenden dreigliedrigen Ring umfasst.

28. Getränk, das ein unsubstituiertes, Methyl- oder Dimethyl-cyclopropylderivat von Citronellol oder Linalool umfasst.

29. Getränk nach einem der Ansprüche 25 bis 28, wobei das Getränk aus der Gruppe von Bier, Malzgetränken, Limonade und Cola ausgewählt ist.

30. Aromatisiertes, oral zugeführtes Produkt, das eine Verbindung umfasst, die durch ein Verfahren erhältlich ist, das das Ersetzen von mindestens einer Kohlenstoff-Kohlenstoff-Doppelbindung in einem Ausgangsmaterial, das aus Citronellol und Linalool ausgewählt ist, durch einen dreigliedrigen Ring umfasst, wobei der dreigliedrige Ring die zwei Kohlenstoffe der Doppelbindung und ein Schwefelatom oder eine C(R)₂-Gruppe, worin jedes R unabhängig voneinander für H, C₁₋₅-Alkyl oder substituiertes C₁₋₅-Alkyl steht und wobei die Substituenten aus der Gruppe von Halogen, Hydroxy, Thiol, Thioether, Amin, Carbonsäure, Ester, Nitro, Cyano, Sulfonsäure, Harnstoff und Thioharnstoff ausgewählt sind, umfasst.

31. Aromatisiertes, oral zugeführtes Produkt nach Anspruch 30, wobei die Verbindung mindestens eine C(R)₂-Gruppe umfasst, wobei mindestens ein R in der C(R)₂-Gruppe Methyl ist.

32. Aromatisiertes, oral zugeführtes Produkt nach Anspruch 30, wobei die Verbindung mindestens einen, ein Schwefelatom umfassenden dreigliedrigen Ring umfasst.

33. Aromatisiertes, oral zugeführtes Produkt, das ein unsubstituiertes, Methyl- oder Dimethyl-cyclopropylderivat von Citronellol oder Linalool umfasst.

34. Verfahren zur Verbesserung, Verstärkung oder Modifizierung des Geruchs einer Parfümzusammensetzung oder eines parfümierten Gegenstands, das die Zugabe von mindestens einer Verbindung umfasst, die durch ein Verfahren erhältlich ist, das das Ersetzen von mindestens einer Kohlenstoff-Kohlenstoff-Doppelbindung in einem Ausgangsmaterial, das aus Citronellol und Linalool ausgewählt ist, durch einen dreigliedrigen Ring umfasst, wobei der dreigliedrige Ring die zwei Kohlenstoffe der Doppelbindung und ein Schwefelatom oder eine C(R)₂-Gruppe, worin jedes R unabhängig voneinander für H, C₁₋₅-Alkyl oder substituiertes C₁₋₅-Alkyl steht und wobei die Substituenten aus der Gruppe von Halogen, Hydroxy, Thiol, Thioether, Amin, Carbonsäure, Ester, Nitro, Cyano, Sulfonsäure, Harnstoff und Thioharnstoff ausgewählt sind, umfasst.

35. Verfahren nach Anspruch 34, wobei die Verbindung mindestens eine C(R)₂-Gruppe umfasst, wobei mindestens ein R in der C(R)₂-Gruppe Methyl ist.

36. Verfahren nach Anspruch 34, wobei die Verbindung mindestens einen, ein Schwefelatom umfassenden dreigliedrigen Ring umfasst.

37. Verfahren zur Verbesserung, Verstärkung oder Modifizierung des Geruchs einer Parfümzusammensetzung oder eines parfümierten Gegenstands, das die Zugabe von mindestens einem unsubstituierten, Methyl- oder Dimethyl-cyclopropylderivat von Citronellol oder Linalool zu der Zusammensetzung oder dem Gegenstand umfasst.

38. Verfahren nach einem der Ansprüche 34 bis 37, wobei die mindestens eine Verbindung im Gemisch mit einem Parfümbestandteil, einem Lösemittel oder einem derzeit auf dem Gebiet der Parfümeriewaren verwendeten Adjuvans vorhanden ist.

39. Verfahren nach einem der Ansprüche 34 bis 37, wobei die mindestens eine Verbindung in einer Menge von mindestens 30 Gew.-% vorhanden ist.

## Revendications

1. Utilisation, en tant qu'aromatisant et/ou fragrance, d'un composé susceptible d'être obtenu par un procédé qui comprend le remplacement d'au moins une double liaison carbone-carbone dans un matériau de départ choisi parmi le citronellol et le linalol par un cycle à trois chaînons, dans lequel le cycle à trois chaînons comprend les deux atomes de carbone de la double liaison et un atome de soufre ou un groupe C(R)₂ dans lequel chaque R est indépendamment H, un alkyle en C₁₋₅ ou alkyle substitué en C₁₋₅, et dans lequel les substituants sont choisis dans le groupe constitué par un halogéno, hydroxy, thiol, thioéther, amine, acide carboxylique, ester, nitro, cyano, acide sulfonique, urée et thiourée.

2. Utilisation selon la revendication 1, dans laquelle le composé comprend au moins un groupe C(R)₂, dans lequel au moins un R dans le groupe C(R)₂ est un méthyle.

3. Utilisation selon la revendication 1, dans laquelle le composé comprend au moins un cycle à trois chaînons comprenant un atome de soufre.

4. Utilisation d'un dérivé cyclopropyle non substitué ou d'un dérivé méthyl- ou diméthyl-cyclopropyle de citronellol ou de linalol en tant qu'aromatisant et/ou fragrance.

5. Utilisation selon l'une quelconque des revendications 1 à 4, dans une composition comprenant un ingrédient parfumant, un solvant, ou un adjuvant d'utilisation courante dans l'art de la parfumerie.

6. Utilisation selon la revendication 5, dans laquelle le composé défini dans les revendications 1 à 4 est présent en une quantité d'au moins 30 % en poids.

7. Utilisation selon la revendication 6, dans laquelle le composé défini dans les revendications 1 à 4 est présent en une quantité d'au moins 60 % en poids.

8. Article parfumé comprenant un composé susceptible d'être obtenu par un procédé qui comprend le remplacement d'au moins une double liaison carbone-carbone dans un matériau de départ choisi parmi le citronellol et le linalol par un cycle à trois chaînons, dans lequel le cycle à trois chaînons comprend les deux atomes de carbone de la double liaison et un atome de soufre ou un groupe C(R)₂ dans lequel chaque R est indépendamment H, un alkyle en C₁₋₅ ou un alkyle substitué en C₁₋₅, et dans lequel les substituants sont choisis dans le groupe constitué par un halogéno, hydroxy, thiol, thioéther, amine, acide carboxylique, ester, nitro, cyano, acide sulfonique, urée et thiourée.

9. Article selon la revendication 8, dans lequel le composé comprend au moins un groupe C(R)₂, dans lequel au moins un R dans le groupe C(R)₂ est un méthyle.

10. Article selon la revendication 8, dans lequel le composé comprend au moins un cycle à trois chaînons comprenant un atome de soufre.

11. Article parfumé comprenant un dérivé cyclopropyle non substitué ou un dérivé méthyl- ou diméthyl-cyclopropyle de citronellol ou de linalol.

12. Article selon l'une quelconque des revendications 8 à 11, comprenant un ingrédient parfumant, un solvant, ou un adjuvant d'utilisation courante dans l'art de la parfumerie.

13. Article selon la revendication 12, dans lequel le composé tel que défini dans les revendications 8 à 11 est présent en une quantité d'au moins 30 % en poids.

14. Article selon la revendication 13, dans lequel le composé tel que défini dans les revendications 8 à 11 est présent en une quantité d'au moins 60 % en poids.

15. Article parfumé selon l'une quelconque des revendications 8 à 11, sous la forme d'un parfum ou d'une eau de Cologne, d'un savon, d'un gel de bain ou de douche, d'un shampooing ou autre produit de soin capillaire, d'une préparation cosmétique, d'un déodorant ou antitranspirant corporel, d'un assainisseur d'air, d'un détergent ou assouplissant pour tissu ou d'un produit de nettoyage domestique universel.

16. Déodorant ou antitranspirant corporel selon la revendication 15.

17. Détergent comprenant un composé susceptible d'être obtenu par un procédé qui comprend le remplacement d'au moins une double liaison carbone-carbone dans un matériau de départ choisi parmi le citronellol et le linalol par un cycle à trois chaînons, dans lequel le cycle à trois chaînons comprend les deux atomes de carbone de la double liaison et un atome de soufre ou un groupe C(R)₂ dans lequel chaque R est indépendamment H, un alkyle en C₁₋₅ ou alkyle substitué en C₁₋₅, et dans lequel les substituants sont choisis dans le groupe constitué par un halogéno, hydroxy, thiol, thioéther, amine, acide carboxylique, ester, nitro, cyano, acide sulfonique, urée et thiourée.

18. Détergent de la revendication 17, dans lequel le composé comprend au moins un groupe C(R)₂, dans lequel au moins un R dans le groupe C(R)₂ est un méthyle.

19. Détergent de la revendication 17, dans lequel le composé comprend au moins un cycle à trois chaînons comprenant un atome de soufre.

20. Détergent comprenant un dérivé cyclopropyle non substitué ou un dérivé méthyl- ou diméthyl-cyclopropyle de citronellol ou de linalol.

21. Composition de blanchiment comprenant un composé susceptible d'être obtenu par un procédé qui comprend le remplacement d'au moins une double liaison carbone-carbone dans un matériau de départ choisi parmi le citronellol et le linalol par un cycle à trois chaînons, dans lequel le cycle à trois chaînons comprend les deux atomes de carbone de la double liaison et un atome de soufre ou un groupe C(R)₂ dans lequel chaque R est indépendamment H, un alkyle en C₁₋₅ ou alkyle substitué en C₁₋₅, et dans lequel les substituants sont choisis dans le groupe constitué par un halogéno, hydroxy, thiol, thioéther, amine, acide carboxylique, ester, nitro, cyano, acide sulfonique, urée et thiourée.

22. Composition de blanchiment selon la revendication 21, dans laquelle le composé comprend au moins un groupe C(R)₂, dans lequel au moins un R dans le groupe C(R)₂ est un méthyle.

23. Composition de blanchiment selon la revendication 21, dans laquelle le composé comprend au moins un cycle à trois chaînons comprenant un atome de soufre.

24. Composition de blanchiment comprenant un dérivé cyclopropyle non substitué ou un dérivé méthyl- ou diméthyl-cyclopropyle de citronellol ou de linalol.

25. Boisson comprenant un composé susceptible d'être obtenu par un procédé qui comprend le remplacement d'au moins une double liaison carbone-carbone dans un matériau de départ choisi parmi le citronellol et le linalol par un cycle à trois chaînons, dans lequel le cycle à trois chaînons comprend les deux atomes de carbone de la double liaison et un atome de soufre ou un groupe C(R)₂ dans lequel chaque R est indépendamment H, un alkyle en C₁₋₅ ou alkyle substitué en C₁₋₅, et dans lequel les substituants sont choisis dans le groupe constitué par un halogéno, hydroxy, thiol, thioéther, amine, acide carboxylique, ester, nitro, cyano, acide sulfonique, urée et thiourée.

26. Boisson selon la revendication 25,dans laquelle le composé comprend au moins un groupe C(R)₂, dans lequel au moins un R dans le groupe C(R)₂ est un méthyle.

27. Boisson selon la revendication 25, dans laquelle le composé comprend au moins un cycle à trois chaînons comprenant un atome de soufre.

28. Boisson comprenant un dérivé cyclopropyle non substitué ou un dérivé méthyl- ou diméthyl-cyclopropyle de citronellol ou de linalol.

29. Boisson selon l'une quelconque des revendications 25 à 28, dans laquelle la boisson est choisie dans le groupe constitué par la bière, la bière forte, la limonade et le cola.

30. Produit aromatisé délivré par voie orale comprenant un composé susceptible être obtenu par un procédé qui comprend le remplacement d'au moins une double liaison carbone-carbone dans un matériau de départ choisi parmi le citronellol et le linalol par un cycle à trois chaînons, dans lequel le cycle à trois chaînons comprend les deux atomes de carbone de la double liaison et un atome de soufre ou un groupe C(R)₂ dans lequel chaque R est indépendamment H, un alkyle en C₁₋₅ ou alkyle substitué en C₁₋₅, et dans lequel les substituants sont choisis dans le groupe constitué par un halogéno, hydroxy, thiol, thioéther, amine, acide carboxylique, ester, nitro, cyano, acide sulfonique, urée et thiourée.

31. Produit aromatisé délivré par voie orale selon la revendication 30, dans lequel le composé comprend au moins un groupe C(R)₂, dans lequel au moins un R dans le groupe C(R)₂ est un méthyle.

32. Produit aromatisé délivré par voie orale selon la revendication 30, dans lequel le composé comprend au moins un cycle à trois chaînons comprenant un atome de soufre.

33. Produit aromatisé délivré par voie orale comprenant un dérivé cyclopropyle non substitué ou un dérivé méthyl- ou diméthyl-cyclopropyle de citronellol ou de linalol.

34. Procédé d'amélioration, d'amplification, ou de modification de l'odeur d'une composition parfumante ou d'un article parfumé comprenant l'addition à la composition ou à l'article d'au moins un composé susceptible d'être obtenu par un procédé qui comprend le remplacement d'au moins une double liaison carbone-carbone dans un matériau de départ choisi parmi le citronellol et le linalol par un cycle à trois chaînons, dans lequel le cycle à trois chaînons comprend les deux atomes de carbone de la double liaison et un atome de soufre ou un groupe C(R)₂ dans lequel chaque R est indépendamment H, un alkyle en C₁₋₅ ou alkyle substitué en C₁₋₅, et dans lequel les substituants sont choisis dans le groupe constitué par un halogéno, hydroxy, thiol, thioéther, amine, acide carboxylique, ester, nitro, cyano, acide sulfonique, urée et thiourée.

35. Procédé selon la revendication 34, dans lequel le composé comprend au moins un groupe C(R)₂, dans lequel au moins un R dans le groupe C(R)₂ est un méthyle.

36. Procédé selon la revendication 34, dans lequel le composé comprend au moins un cycle à trois chaînons comprenant un atome de soufre.

37. Procédé d'amélioration, d'amplification, ou de modification de l'odeur d'une composition parfumante ou d'un article parfumé comprenant l'addition à la composition ou à l'article d'au moins un dérivé cyclopropyle non substitué ou dérivé méthyl- ou diméthyl-cyclopropyle de citronellol ou de linalol.

38. Procédé selon l'une quelconque des revendications 34 à 37, dans lequel le au moins un composé est présent dans un mélange avec un ingrédient parfumant, un solvant, ou un adjuvant d'utilisation courante dans l'art de la parfumerie.

39. Procédé selon l'une quelconque des revendications 34 à 37, dans lequel le au moins un composé est présent en une quantité d'au moins 30 % en poids.
